# EUROPEAN PATENT APPLICATION

(11) **EP 4 317 420 A1**
(43) Date of publication of application: **07.02.2024**
(21) Application number: 22781580.0
(22) Date of filing: 29.03.2022
(51) Int. Cl.: C12N 5/0775

(54) **MEDIUM COMPOSITION FOR CULTURING MUSCLE STEM CELL COMPRISING CURCUMIN LONGA, GLYSIN, OR INSULIN FOR PROLIFERATION OF MUSCLE STEM CELL**

(30) Priority: 29.03.2021 KR 20210040443; 29.03.2021 KR 20210040444; 29.03.2021 KR 20210040445
(71) Applicant: Hyupsung University Industry-Academic Cooperation Foundation, Hwaseong-si, Gyeonggi-do 18330 (KR); Danagreen Co., Ltd., Seoul 06570 (KR)
(72) Inventor: HAN, Jiyou, Seoul 04393 (KR); LEE, Jun Hyoung, Suwon-si, Gyeonggi-do 16382 (KR); JEE, Hyeongun, Seoul 06561 (KR); PARK, Jongbong, Chuncheon-si, Gangwon-do 24318 (KR); CHOI, Kyung Ah, Seoul 06571 (KR); KANG, Kyojin, Seoul 02147 (KR)
(74) Representative: Vossius & Partner Patentanwälte Rechtsanwälte mbB
(86) International application number: PCT/KR2022/004437
(87) International publication number: WO 2022/211461

(57) **Abstract**

Provided is a culture medium composition including turmeric, glycine, or insulin for culturing muscle stem cells for proliferation of muscle stem cells. According to the culture medium composition including turmeric, glycine, or insulin muscle stem cells for culturing muscle stem cells according to an aspect, the proliferation and differentiation abilities of cells do not degrade even when culturing muscle stem cells in vitro, resulting in effects of enabling mass culture of muscle stem cells. Accordingly, the culture medium composition is economical by effectively replacing a culture medium containing expensive bFGF, and since appropriate time of differentiation can be chosen when culturing muscle stem cells, the muscle stem cells can be differentiated when needed, thereby producing an effect of improving the quality of in vitro meat.

## Description

### Technical Field

The present disclosure relates to a culture medium composition including turmeric, glycine, or insulin for culturing muscle stem cells used for proliferation of muscle stem cells.

### Background Art

Stem cells refer to cells that have not yet differentiated and can become other cells, and may have ability to self-reproduce, i.e., ability to continuously produce cells identical to themselves, in an undifferentiated state, and ability to differentiate into specific cells when cultured under suitable conditions.

Turmeric (*Curcumin longa*) is a perennial herb belonging to the ginger family under the ginger order that has been used as oriental medicine in the traditional herbal medicine, and it has been used as an edible herb, a food coloring, or a cosmetic ingredient [Foods. 2017 Oct; 6(10): 92]. Also, the turmeric has an anti-cancer effect, an anti-inflammatory effect, an antioxidant effect, or the like.

The term "in vitro meat" refers to edible meat obtained by collecting cells from living animals and proliferating the cells by using cell engineering techniques, and is regarded as a field of cellular agriculture in which meat is obtained without undergoing livestock breeding. Regarding the production of such in vitro meat, methods of preparing in vitro meat through in vitro culture may be commonly practiced.

When culturing the muscle stem cells in vitro, basic fibroblast growth factor (bFGF) needs to be continuously added to a culture or support medium to maintain the undifferentiated state, which is very costly. Therefore, it is necessary to develop a material that can effectively replace bFGF when culturing the stem cells in vitro.

While carrying out research based on this, the inventors of the present disclosure found that a culture medium composition including turmeric, glycine, or insulin was able to maintain the undifferentiated state of muscle stem cells even without additional supply of bFGF, and to promote the proliferation of muscle stem cells. Accordingly, the inventors found that turmeric, glycine, or insulin can effectively replace bFGF, thereby completing the present disclosure

### Disclosure

### Technical Problem

One aspect is to provide a culture medium composition including turmeric, glycine, or insulin for culturing muscle stem cells.

Another aspect is to provide a method of culturing muscle stem cells, the method including culturing muscle stem cells in the culture medium composition.

Another aspect is to provide a method of culturing in vitro meat, the method including preparing in vitro meat by treating the culture medium composition with isolated muscle stem cells.

Another aspect is to provide in vitro meat prepared by the method of culturing in vitro meat.

Another aspect is to provide use of turmeric, glycine, or insulin for culturing muscle stem cells.

### Technical Solution

One aspect provides a culture medium composition including turmeric, glycine, or insulin for culturing muscle stem cells.

The term "turmeric (*Curcuma longa*)" as used herein refers to a perennial herb belonging to the ginger family under the ginger order that has been used as oriental medicine in the traditional herbal medicine.

In an embodiment, the turmeric may be a turmeric extract or a fraction thereof. The turmeric extract or a fraction thereof may include curcumin, demethoxycurcumin, bisdemethoxycurcumin, or the like.

The turmeric extract may be obtained by extraction from various organs of native, hybrid, and mutated plants. For example, it may be obtained by extraction from roots, stems, leaves, flowers, fruit bodies, fruit skins, or plant tissue cultures.

Regarding extraction methods of obtaining the extract, the extract may be prepared according to known physical, chemical, or enzymatic extraction methods commonly used in the technical field to which the present disclosure pertains. In detail, commonly used extraction methods, such as a cold precipitation method, a heat extraction method, an ultrasonic extraction, a filtration method, a pressure extraction method, a reflux extraction method, a supercritical extraction method, an electric extraction method, a steam distillation method, an organic solvent extraction, a hot-water extraction method, etc., may be used, and commonly used extraction devices, ultrasonic pulverizers, fractionators may be used.

In an embodiment, the extract may be obtained by extraction using water, alcohol, or a mixture thereof as a solvent. The alcohol may be C1-C6 alcohol, such as C1-C4 alcohol. The C1-C6 alcohol may be methanol, ethanol, propanol, isopropanol, 1,3-propanediol, butanol, pentanol, hexanol, or butylene glycol.

In an embodiment, the extraction may include adding an extraction solvent at 3 times to 10 times (by volume or weight), e.g., 3 times to 7 times (by volume or weight), 3 times to 5 times (by volume or weight), 5 times to 10 times (by volume or weight), or 4 times to 10 times (by volume or weight), as much as each extract.

In an embodiment, the solvent may be a mixture of water and alcohol, that is, an aqueous alcohol solution such as an aqueous ethanol solution. The alcohol concentration in the aqueous alcohol solution may be 1 (v/v)% to 99.5 (v/v)%, 10 (v/v)% to 99.5 (v/v)%, 1 (v/v)% to 70(v/v)%, 1 (v/v)% to 40 (v/v)%, 5 (v/v)% to 25 (v/v)%, 7 (v/v)% to 20 (v/v)%, 5 (v/v)% to 25 (v/v)%, or 10 (v/v)% to 20 (v/v)%.

Regarding the fraction of the turmeric extract, the turmeric extract obtained by the aforementioned method may be fractionated using a known method commonly used in the technical field to which the present disclosure pertains through any one or more fractionation solvents of water, ethyl acetate, and n-butanol.

The term "muscle stem cells" as used herein refers to cells having muscle stem cell characteristics including all abilities to proliferate without transformation, to proliferate infinitely, to self-renew, and to differentiate into muscles. Any cells showing self-renewal ability or infinite proliferation ability and muscle differentiation ability may be used without limitation. The self-renewal ability and differentiation ability into muscles may be confirmed with markers. Also, the muscle stem cells may be, for example, cells that exhibit expression of CD29, CD56, Oct4, Nanog, or Pax7, along with the self-renewal ability. Types or origins of the muscle stem cells are not limited as long as the cells have the differentiation ability and self-renewal ability. The muscle stem cells may be derived from, for example, mammals, humans, monkeys, pigs, horses, cows, chickens, ducks, sheep, dogs, cats, mice, rabbits, etc. In an embodiment, the muscle stem cells may be bovine or chicken muscle stem cells.

The muscle stem cells may be cells that express Pax7 or MyoD in a process of being induced into myoblasts.

The term "myoblasts" as used herein refers to precursor cells during a process of differentiating into muscle cells, and as in the case of myoblasts, the term also refers to cells that proliferate land differentiate into muscle cells (e.g., myogenic cells or myocytes). Th term "muscle cells" as used herein refers to cells capable of constituting muscle tissues generated from myoblasts through a differentiation process of myoblasts.

The term "cell culture" as used in the present specification refers to a process of artificially growing living cells in vitro under controlled conditions. In addition, the term also refers to cells grown and proliferated by spreading a suspension, in which a portion of an individual's tissue is aseptically detached and intercellular linking materials are dissolved and liberated with an enzyme, on a flat bottom of a bottle or a culture dish such as a Petri dish.

The term "culture media" as used herein refers to a material that supports the growth and survival of cells, including stem cells, in vitro.

The culture medium composition is not particularly limited as long as it can be used for cell culture by adding turmeric, glycine, or insulin thereto. For example, the culture medium composition may be prepared by adding turmeric, glycine, or insulin to one or more selected from Dulbecco's modified eagle's medium (DMEM), minimal essential medium (MEM), basal medium eagle (BME), RPMI 1640, F-10, F-12, DMEM/F12, α-minimal essential medium (α-MEM), Glasgow's minimal essential medium (G-MEM), Iscove's modified Dulbecco's medium (IMDM), MacCoy's 5A medium, AmnioMax complete medium, AminoMax ± complete medium, endothelial basal medium (EBM), Chang's Medium, MesenCult-XF, Dulbecco's modified Eagle's medium/ high glucose (DMEM/HG) medium, and MCDB + Dulbecco's modified Eagle's medium/ low glucose (MCDB+DMEM/LG) medium.

In addition, the culture medium may include amino acids, vitamins, inorganic salts, and other ingredients. For example, the amino acids may include a non-essential amino acid and L-glutamine, the vitamins may include vitamin B12, the inorganic salts may include a trace ingredient (e.g., CuSO₄5H₂O, Fe(NO₃)₃9H₂O, ZnSO₄, etc.), phosphoenol pyruvate, momoethanolamine, sodium selenite, and sodium bicarbonate, and the other ingredients may include D-glucose, linoleic acid, lipoic acid, sodium pyruvate, hypoxantine Na, putrescine HCl, and polyamine solution.

The culture may refer to growth and proliferation.

The term "growth and proliferation" as used herein refers to an increase in the number of cells. The culture may refer to undifferentiated proliferation. The undifferentiated proliferation refers that stem cells proliferate into cells having the same properties as the original cells, i.e., cells having potency and self-renewal ability, without being differentiated into specific cells. The term "differentiation" as used herein refers to a phenomenon in which cells become specialized in structure or function during the growth by division and proliferation. That is, the term refers to a change in the form or function of cells, tissues, or the like of living organisms to perform tasks given thereto. Measuring or determining the degree of differentiation into specific cell types may be performed by methods well known in the art. In addition, the differentiation may be confirmed by examining cell morphology using an optical microscope or a confocal microscope while measuring changes in cell surface markers (e.g., by staining cells with tissue-specific or cell-label-specific antibodies) and cell morphology (e.g., nuclear ratio/cytoplasm ratio) using techniques such as flow cytometry or immunocytochemistry, or by measuring changes in gene expression using techniques well known in the art, such as polymerase chain reaction (PCR) and gene-expression profile.

In an embodiment, the culture medium composition may be able to maintain muscle stem cells in an undifferentiated state. In this regard, the culture medium composition may be able to efficiently proliferate muscle stem cells while maintaining the muscle stem cells in an undifferentiated state.

In an embodiment, the culture medium may not include basic fibroblast growth factor (bFGF).

The term "bFGF" as used herein refers to a protein belonging to the FGF family that is involved in cell proliferation and cell differentiation and that functions as a mitogenic factor, an angiogenic factor, a bone-forming factor, and a neurotrophic factor. The term is also called FGF2, and mainly activates receptor proteins including FGFR 1b, FGFR 1c, FGFR 2c, FGFR 3c, and FGFR 4c, and it is known to strongly activate specifically FGFR 1c and FGFR 3c. The bFGF may serve to enhance the proliferation and differentiation ability of stem cells.

The term "substantially does not comprise" or "does not comprise" refers that the bFGF is included to the extent that it does not affect the physical properties, activity, or pharmacological activity of the culture medium composition, or that the bFGF is not included at all. For example, the bFGF may be included in an amount of 0.02 wt% or less, 0.01 wt% or less, or 0.005 wt% or less, with respect to the amount of the culture medium composition for culturing muscle stem cells, or may not be included at all.

In an embodiment, the culture medium composition may include curcumin in a concentration of 0.01 µm to 100 µm. For example, the culture medium composition may include curcumin in a concentration of 0.01 µm to 100 µm, curcumin in a concentration of 0.05 µm to 100 µm, curcumin in a concentration of 0.1 µm to 100 µm, curcumin in a concentration of 0.1 µm to 50 µm, curcumin in a concentration of 0.1 µm to 40 µm, curcumin in a concentration of 0.1 µm to 30 µm, curcumin in a concentration of 0.1 µm to 20 µm, curcumin in a concentration of 0.5 µm to 100 µm, curcumin in a concentration of 0.5 µm to 50 µm, curcumin in a concentration of 0.5 µm to 40 µm, curcumin in a concentration of 0.5 µm to 30 µm, curcumin in a concentration of 0.5 µm to 20 µm, curcumin in a concentration of 0.5 µm to 10 µm, curcumin in a concentration of 1 µm to 100 µm, curcumin in a concentration of 1 µm to 50 µm, curcumin in a concentration of 1 µm to 40 µm, curcumin in a concentration of 1 µm to 30 µm, curcumin in a concentration of 1 µm to 20 µm, curcumin in a concentration of 1 µm to 10 µm, curcumin in a concentration of 1 µm to 9 µm, or curcumin in a concentration of 1 µm to 8 µm.

In an embodiment, the culture medium composition may include curcumin in a concentration of at least 8 µm.

In an embodiment, the culture medium composition may include glycine in a concentration of 0.01 µm to 1,000 µm. For example, the culture medium composition may include glycine in a concentration of 0.1 mM to 1,000 mM, glycine in a concentration of 1 mM to 1,000 mM, glycine in a concentration of 10 mM to 1,000 mM, glycine in a concentration of 10 mM to 500 mM, glycine in a concentration of 50 mM to 500 mM, glycine in a concentration of 50 mM to 400 mM, glycine in a concentration of 50 mM to 300 mM, glycine in a concentration of 50 mM to 200 mM, glycine in a concentration of 50 mM to 150 mM, glycine in a concentration of 100 mM to 1,000 mM, glycine in a concentration of 100 mM to 500 mM, glycine in a concentration of 100 mM to 400 mM, glycine in a concentration of 100 mM to 300 mM, glycine in a concentration of 100 mM to 200 mM, or glycine in a concentration of 100 mM to 150 mM.

In an embodiment, the culture medium composition may include glycine in a concentration of at least 100 mM.

In an embodiment, the culture medium composition may include insulin in a concentration of 0.01 µm to 100 µm. For example, the culture medium composition may include insulin in a concentration of 0.01 µm to 100 µm, insulin in a concentration of 0.05 µm to 100 µm, insulin in a concentration of 0.1 µm to 100 µm, insulin in a concentration of 0.1 µm to 50 µm, insulin in a concentration of 0.1 µm to 40 µm, insulin in a concentration of 0.1 to 30 µm, insulin in a concentration of 0.1 µm to 20 µm, insulin in a concentration of 0.5 µm to 100 µm, insulin in a concentration of 0.5 µm to 50 µm, insulin in a concentration of 0.5 µm to 40 µm, insulin in a concentration of 0.5 µm to 30 µm, insulin in a concentration of 0.5 µm to 20 µm, insulin in a concentration of 0.5 µm to 10 µm, insulin in a concentration of 1 µm to 100 µm, insulin in a concentration of 1 µm to 50 µm, insulin in a concentration of 1 µm to 40 µm, insulin in a concentration of 1 µm to 30 µm, insulin in a concentration of 1 µm to 20 µm, or insulin in a concentration of 1 µm to 10 µm.

In an embodiment, the culture medium composition may include insulin in a concentration of at least 10 µm.

Another aspect of the disclosure provides a method of culturing muscle stem cells, the method including culturing isolated muscle stem cells in a medium for culturing muscle stem cells including turmeric, glycine, or insulin.

The mammal may include, but is not limited thereto, a human, a cow, a pig, a dog, a cat, a horse, a monkey, etc.

Methods of the culturing may include passage culture.

The term "passage culture" as used herein is one of cell proliferation methods, and refers to transplanting cells into a fresh medium periodically, every two days, to preserve the cells and continue the cell lineage. The term "passage" as used herein refers to growth and proliferation of stem cells in a culture vessel from initial seeding, until confluence, i.e., the time when cells actively grow, is reached in the same culture vessel. Method of cell culture and passage culture may be performed by conventional culture methods known in the art.

According to the method of culturing cells by using the culture medium composition, the cells may be obtained by proliferating and growing muscle stem cells that maintain the unique characteristics thereof in large quantities.

Another aspect provides a method of culturing in vitro meat, the method including preparing in vitro meat by treating the culture medium composition with isolated muscle stem cells derived from a non-human animal.

The term "in vitro meat" as used herein refers to edible meat obtained by collecting cells from living animals and proliferating the cells using cell engineering technology. In an embodiment, the in vitro meat may be, but is not limited thereto, edible meat obtained by collecting cells from cows or chickens and proliferating the cells using cell engineering technology.

The in vitro meat may also be prepared as a synthetic food product, and the synthetic food product may further include, in addition to the in vitro meat, a mineral, a vitamin, a supplementary-vitamin, an essential fatty acid, an essential amino acid, an enzyme, an antioxidant, or a combination of two or more thereof.

In addition, the synthetic food product including the in vitro meat may further include a flavoring agent, a flavor enhancer, a sweetening agent, a colorant, a color former, a bleaching agent, a preservative, a sterilizer, an antioxidant, a leavening agent, a coagulant, or a thickener.

The in vitro meat may be specifically processed or prepared in the form of processed meat products, and the processed meat products may include, for example, sterilized meat products, hams, pressed hams, mixed pressed hams, sausages, mixed sausages, dried sausages (dried mixed sausages), half-dried sausages (half-dried mixed sausages), heat frozen sausages, bacons, dry-cured meat, seasoned meat, processed ground meat products, packaged meat, or other meat products, but embodiments of the present disclosure are not limited thereto.

The method of preparing the in vitro meat may include: promoting proliferation of the muscle stem cells by treating isolated muscle stem cells derived from a non-human animal with the culture medium composition while maintaining undifferentiation of the muscle stem cells; and inducing differentiation of the muscle stem cells into myoblasts by treating isolated muscle stem cells derived from a non-human animal with the culture medium composition while promoting the proliferation of the muscle stem cells.

In an embodiment, the promoting of proliferation of the muscle stem cells while maintaining undifferentiation of the muscle stem cells may include treating the muscle stem cells with a culture medium composition including curcumin in a concentration of 0.01 µm to 100 µm. For example, the culture medium composition used in the treating of the muscle stems may include curcumin in a concentration of 0.01 µm to 100 µm, curcumin in a concentration of 0.05 µm to 100 µm, curcumin in a concentration of 0.1 µm to 100 µm, curcumin in a concentration of 0.1 µm to 50 µm, curcumin in a concentration of 0.1 µm to 40 µm, curcumin in a concentration of 0.1 µm to 30 µm, curcumin in a concentration of 0.1 µm to 20 µm, curcumin in a concentration of 0.5 µm to 100 µm, curcumin in a concentration of 0.5 µm to 50 µm, curcumin in a concentration of 0.5 µm to 40 µm, curcumin in a concentration of 0.5 µm to 30 µm, curcumin in a concentration of 0.5 µm to 20 µm, curcumin in a concentration of 0.5 µm to 10 µm, curcumin in a concentration of 1 µm to 100 µm, curcumin in a concentration of 1 µm to 50 µm, curcumin in a concentration of 1 µm to 40 µm, curcumin in a concentration of 1 µm to 30 µm, curcumin in a concentration of 1 µm to 20 µm, curcumin in a concentration of 1 µm to 10 µm, curcumin in a concentration of 1 µm to 9 µm, or curcumin in a concentration of 1 µm to 8 µm.

In an embodiment, the promoting of proliferation of the muscle stem cells while maintaining undifferentiation of the muscle stem cells may include treating the muscle stem cells with a culture medium composition including glycine in a concentration of 0.1 µm to 1,000 µm. For example, the culture medium composition used in the treating of the muscle stems may include glycine in a concentration of 0.1 mM to 1,000 mM, glycine in a concentration of 1 mM to 1,000 mM, glycine in a concentration of 10 mM to 1,000 mM, glycine in a concentration of 10 mM to 500 mM, glycine in a concentration of 50 mM to 500 mM, glycine in a concentration of 50 mM to 400 mM, glycine in a concentration of 50 mM to 300 mM, glycine in a concentration of 50 mM to 200 mM, glycine in a concentration of 50 mM to 150 mM, glycine in a concentration of 100 mM to 1,000 mM, glycine in a concentration of 100 mM to 500 mM, glycine in a concentration of 100 mM to 400 mM, glycine in a concentration of 100 mM to 300 mM, glycine in a concentration of 100 mM to 200 mM, or glycine in a concentration of 100 mM to 150 mM.

In an embodiment, the promoting of proliferation of the muscle stem cells while maintaining undifferentiation of the muscle stem cells may include treating the muscle stem cells with a culture medium composition including insulin in a concentration of 0.01 µm to 100 µm. For example, the culture medium composition used in the treating of the muscle stems may include insulin in a concentration of 0.01 µm to 100 µm, insulin in a concentration of 0.05 µm to 100 µm, insulin in a concentration of 0.1 µm to 100 µm, insulin in a concentration of 0.1 µm to 50 µm, insulin in a concentration of 0.1 µm to 40 µm, insulin in a concentration of 0.1 to 30 µm, insulin in a concentration of 0.1 µm to 20 µm, insulin in a concentration of 0.5 µm to 100 µm, insulin in a concentration of 0.5 µm to 50 µm, insulin in a concentration of 0.5 µm to 40 µm, insulin in a concentration of 0.5 µm to 30 µm, insulin in a concentration of 0.5 µm to 20 µm, insulin in a concentration of 0.5 µm to 10 µm, insulin in a concentration of 1 µm to 100 µm, insulin in a concentration of 1 µm to 50 µm, insulin in a concentration of 1 µm to 40 µm, insulin in a concentration of 1 µm to 30 µm, insulin in a concentration of 1 µm to 20 µm, or insulin in a concentration of 1 µm to 10 µm.

In an embodiment, the inducing of differentiation of the muscle stem cells into myoblasts by treating isolated muscle stem cells derived from a non-human animal with the culture medium composition while promoting the proliferation of the muscle stem cells may include treating with the culture medium composition including curcumin in a concentration of at least 8 µm.

In an embodiment, the inducing of differentiation of the muscle stem cells into myoblasts by treating isolated muscle stem cells derived from a non-human animal with the culture medium composition while promoting the proliferation of the muscle stem cells may include treating with the culture medium composition including glycine in a concentration of at least 100 mM.

In an embodiment, the inducing of differentiation of the muscle stem cells into myoblasts by treating isolated muscle stem cells derived from a non-human animal with the culture medium composition while promoting the proliferation of the muscle stem cells may include treating with the culture medium composition including insulin in a concentration of at least 10 µm.

According to the method of preparing the in vitro meat by using the culture medium composition, the undifferentiation of the muscle stem cells may be promoted while maintaining the undifferentiation of the muscle stem cell, and the differentiation time of the muscle stem cells may be appropriately selected. Thus, by differentiating the muscle stem cells when needed, the quality of the in vitro meat may be improved.

Another aspect provides in vitro meat prepared by the method of culturing in vitro meat.

The in vitro meat is the same as described above.

Another aspect provides use of turmeric, glycine, or insulin for culturing muscle stem cells.

The turmeric, glycine, or insulin may be included in the cell culture medium.

The muscle stem cell, turmeric, glycine, insulin, and medium are the same as described above.

### Advantageous Effects

According to a culture medium composition including turmeric, glycine, or insulin for culturing muscle stem cells, the proliferation ability and differentiation ability of cells are not reduced even when the muscle stem cells cultured in vitro, and thus the muscle stem cells may be mass-cultured. Accordingly, the culture medium composition is economical by effectively replacing a culture medium containing expensive bFGF, and since appropriate time of differentiation can be chosen when culturing muscle stem cells, the quality of in vitro meat may be improved by differentiating the muscle stem cells when needed.

### Description of Drawings

FIG. 1 is a graph showing effects of a culture medium supplemented with curcumin and bFGF on proliferation of muscle stem cells.
FIG. 2 is a graph showing effects of a bFGF-free curcumin culture medium on proliferation of muscle stem cells.
FIG. 3 is a graph showing an expression level of Oct4, which is a muscle stem cell marker, according to a concentration of curcumin in a culture medium supplemented with curcumin and bFGF.
FIG. 4 is a graph showing an expression level of Nanog, which is a muscle stem cell marker, according to a concentration of curcumin in a culture medium supplemented with curcumin and bFGF.
FIG. 5 is a graph showing an expression level of Pax7, which is a myoblast marker, according to a concentration of curcumin in a culture medium supplemented with curcumin and bFGF.
FIG. 6 is a graph showing an expression level of Oct4, which is a muscle stem cell marker, according to a concentration of curcumin in a bFGF-free curcumin culture medium.
FIG. 7 is a graph showing an expression level of Nanog, which is a muscle stem cell marker, according to a concentration of curcumin in a bFGF-free curcumin culture medium.
FIG. 8 is a graph showing an expression level of Pax7, which is a myoblast marker, according to a concentration of curcumin in a bFGF-free curcumin culture medium.
FIG. 9 is a graph showing effects of a culture medium supplemented with glycine and bFGF on proliferation of muscle stem cells.
FIG. 10 is a graph showing effects of a bFGF-free glycine culture medium on proliferation of muscle stem cells.
FIG. 11 is a graph showing an expression level of Oct4, which is a muscle stem cell marker, according to a concentration of glycine in a culture medium supplemented with glycine and bFGF.
FIG. 12 is a graph showing an expression level of Nanog, which is a muscle stem cell marker, according to a concentration of glycine in a culture medium supplemented with glycine and bFGF.
FIG. 13 is a graph showing an expression level of Pax7, which is a myoblast marker, according to a concentration of glycine in a culture medium supplemented with glycine and bFGF.
FIG. 14 is a graph showing an expression level of Oct4, which is a muscle stem cell marker, according to a concentration of glycine in a bFGF-free glycine culture medium.
FIG. 15 is a graph showing an expression level of Nanog, which is a muscle stem cell marker, according to a concentration of glycine in a bFGF-free glycine culture medium.
FIG. 16 is a graph showing an expression level of Pax7, which is a myoblast marker, according to a concentration of glycine in a bFGF-free glycine culture medium.
FIG. 17 is a graph showing effects of a culture medium supplemented with insulin and bFGF on proliferation of muscle stem cells.
FIG. 18 is a graph showing effects of a bFGF-free insulin culture medium on proliferation of muscle stem cells.
FIG. 19 is a graph showing an expression level of Oct4, which is a muscle stem cell marker, according to a concentration of insulin in a culture medium supplemented with insulin and bFGF.
FIG. 20 is a graph showing an expression level of Nanog, which is a muscle stem cell marker, according to a concentration of insulin in a culture medium supplemented with insulin and bFGF.
FIG. 21 is a graph showing an expression level of Pax7, which is a myoblast marker, according to a concentration of insulin in a culture medium supplemented with insulin and bFGF.
FIG. 22 is a graph showing an expression level of Oct4, which is a muscle stem cell marker, according to a concentration of insulin in a bFGF-free insulin culture medium.
FIG. 23 is a graph showing an expression level of Nanog, which is a muscle stem cell marker, according to a concentration of insulin in a bFGF-free insulin culture medium.
FIG. 24 is a graph showing an expression level of Pax7, which is a myoblast marker, according to a concentration of insulin in a bFGF-free insulin culture medium.

### BEST MODE

### Mode for Invention

Hereinafter, the present disclosure will be described in more detail with reference to Examples below. However, these Examples are for illustrative purposes only, and the scope of the present disclosure is not intended to be limited by these Examples.

### Example 1. Preparation of curcumin-containing culture medium

A DMEM basal medium was prepared by adding 5 ml of penicillin-streptomycin (10,000 U/mL) to 500 ml of DMEM (Gibco #10313039). Curcumin was added to the DMEM basal medium to prepare a culture medium supplemented with curcumin. In order to confirm the effect of maintaining differentiation ability according to the concentration of curcumin, a culture medium was prepared by varying the concentrations of curcumin (0.1 µm, 1 µm, 5 µm, and 8 µm, respectively).

### Example 2. Preparation of glycine-containing medium

A DMEM basal medium was prepared by adding 5 ml of penicillin-streptomycin (10,000 U/mL) to 500 ml of DMEM (Gibco #10313039). Glycine was added to the DMEM basal medium to prepare a culture medium supplemented with glycine. In order to confirm the effect of maintaining differentiation ability according to the concentration of glycine, a culture medium was prepared with different concentrations of glycine (1 mM, 10 mM, 50 mM, 100 mM, and 150 mM, respectively).

### Example 3. Preparation of insulin-containing medium

A DMEM basal medium was prepared by adding 5 ml of penicillin-streptomycin (10,000 U/mL) to 500 ml of DMEM (Gibco #10313039). Curcumin was added to the DMEM basal medium to prepare a culture medium supplemented with insulin. In order to confirm the effect of maintaining differentiation ability according to the concentration of insulin, a culture medium was prepared with different concentrations of insulin (0.1 µm, 1 µm, 5 µm, 8 µm, and 10 µm, respectively).

### Experimental Example 1. Confirmation of effects of curcumin-containing culture medium on proliferation of muscle stem cells

### Experimental Example 1.1 Conformation of culture medium supplemented with curcumin and bFGF on proliferation of muscle stem cells

5 % FBS, 8 ng/ml of bFGF, and 8 µm of curcumin were added to the DMEM basal medium of Example 1, and effects of proliferating muscle stem cells were confirmed.

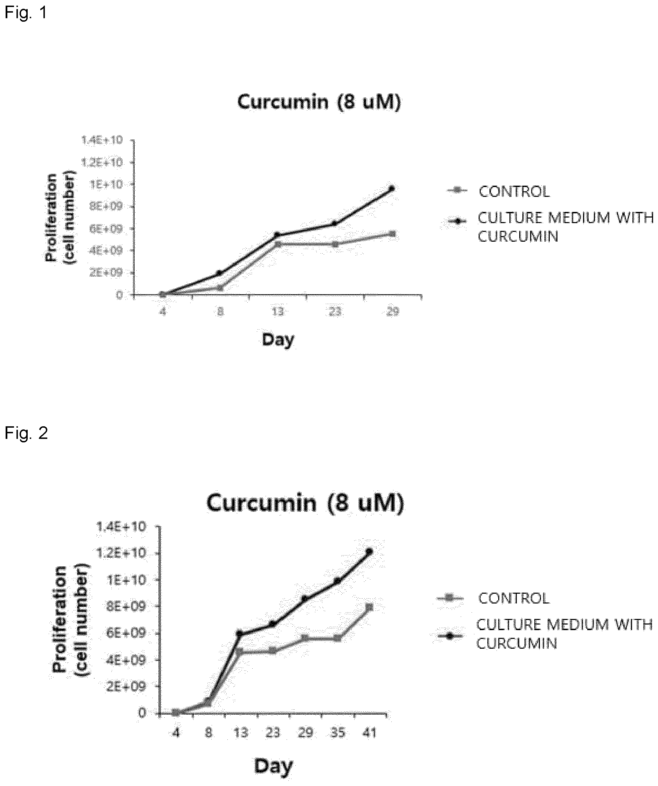

In detail, first, bovine rump meat was treated with trypsin, and then primary-cultured to obtain bovine muscle stem cells. Afterwards, the muscle stem cells were cultured in the DMEM basal medium supplemented with 5% FBS, 8 µm of curcumin, and 8 ng/ml of bFGF, and the proliferation of the muscle stem cells was measured. Next, the results are shown in FIG. 1. As a control, a DMEM basal medium supplemented with 5 % FBS + 8 ng/ml of bFGF was used.

FIG. 1 is a graph showing effects of the culture medium supplemented with curcumin and bFGF on the proliferation of muscle stem cells.

As shown in FIG. 1, it was confirmed that, when the muscle cells were cultured for 29 days, the cell proliferation rate in the culture medium supplemented with 8 µm of curcumin was increased by about 1.5 times or more compared to that in the basal medium.

### Experimental Example 1.2 Confirmation of bFGF-free curcumin culture medium on proliferation of muscle stem cells

5 % FBS and 8 µm of curcumin were added to the DMEM basal medium of Example 1, and effects of proliferating muscle stem cells were confirmed.

In detail, first, bovine rump meat was treated with trypsin, and then primary-cultured to obtain bovine muscle stem cells. Afterwards, the muscle stem cells were cultured in the DMEM basal medium supplemented with 5% FBS and 8 µM of curcumin, and the proliferation of the muscle stem cells was measured. Next, the results are shown in FIG. 2. As a control, a DMEM basal medium supplemented with 5 % FBS was used.

FIG. 2 is a graph showing effects of the bFGF-free curcumin culture medium on the proliferation of muscle stem cells.

As shown in FIG. 2, it was confirmed that, when the muscle cells were cultured for 41 days, the cell proliferation rate in the culture medium supplemented with 8 µm of curcumin was increased by about 1.5 times or more compared to that in the basal medium.

Referring to the results above, it was confirmed that, regardless of the presence of bFGF, curcumin could act as a proliferation promoter for muscle stem cells.

### Experimental Example 2. Confirmation of effects of curcumin-containing culture medium on maintaining differentiation ability of muscle stem cells

### Experimental Example 2.1 Conformation of culture medium supplemented with curcumin and bFGF on maintaining differentiation ability of muscle stem cells

5 % FBS, 8 ng/ml of bFGF, and curcumin were added to the DMEM basal medium of Example 1, and effects of maintaining differentiation ability of muscle stem cells were confirmed.

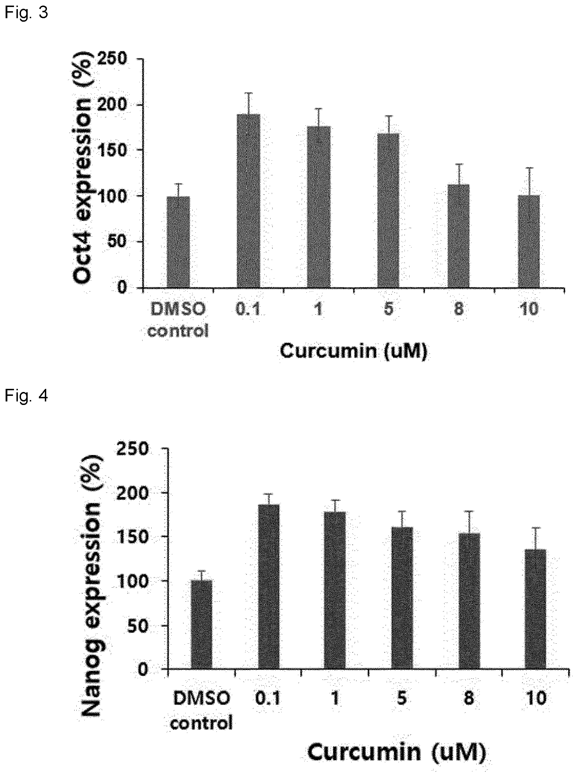

In detail, first, bovine rump meat was treated with trypsin, and then primary-cultured to obtain bovine muscle stem cells. Afterwards, a culture medium supplemented with curcumin and bFGF was prepared by varying the concentration of curcumin (0.1 µm, 1 µm, 5 µm, 8 µm, and 10 8 µm, respectively) in a DMEM basal medium supplemented with 5 % FBS and 8 ng/ml of bFGF, and the muscle stem cells were cultured through passage culture twice. In addition, the expression levels of Oct4 and Nanog as muscle stem cell markers and Pax7 as a myoblast marker in the culture medium were confirmed through qPCR, and the results are shown in FIGS. 3 to 5, respectively.

FIG. 3 is a graph showing the expression level of Oct4 as a muscle stem cell marker according to the concentration of curcumin in the culture medium supplemented with curcumin and bFGF.

FIG. 4 is a graph showing the expression level of Nanog as a muscle stem cell marker according to the concentration of curcumin in the culture medium supplemented with curcumin and bFGF.

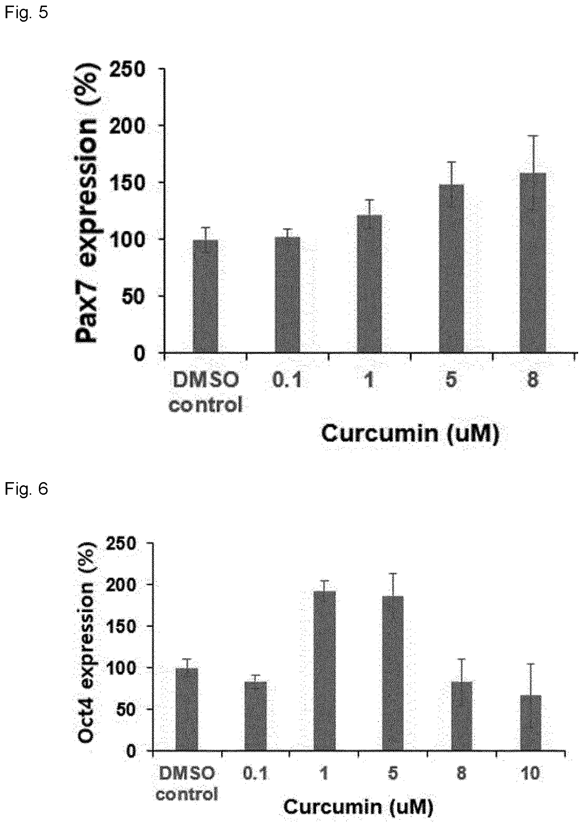

FIG. 5 is a graph showing the expression level of Pax7 as a myoblast marker according to the concentration of curcumin in the culture medium supplemented with curcumin and bFGF.

As shown in FIG. 3, the expression of Oct 4 increased in the culture medium supplemented with curcumin and bFGF, confirming that the differentiation ability of the muscle stem cells was effectively maintained. In particular, it was confirmed that the expression of Oct4 in the culture medium supplemented with curcumin in the concentration of 0.1 µm to 5 µm increased by about twice the expression of Oct4 in the curcumin-free culture medium control (i.e., DMSO control). Also, it was confirmed that, when the concentration of curcumin was 8 µm or more, the expression of Oct4 significantly decreased.

As shown in FIG. 4, the expression of Nanog increased in the culture medium supplemented with curcumin and bFGF, confirming that the differentiation ability of the muscle stem cells was effectively maintained. In particular, it was confirmed that the expression of Nanog in the culture medium supplemented with curcumin in the concentration of 0.1 µm and 1 µm increased by about twice the expression of Nanog in the curcumin-free culture medium control (i.e., DMSO control). Also, it was confirmed that, when the concentration of curcumin was 5 µm or more, the expression of Nanog significantly decreased.

As shown in FIG. 5, the expression of Pax7 increased in proportion to the concentration of curcumin in the culture medium supplemented with curcumin and bFGF.

Referring to the results above, it was confirmed that the differentiation ability of muscle stem cells could be maintained when the muscle stem cells were cultured in the culture medium supplemented with curcumin. Also, it was confirmed that, in the culture medium supplemented with curcumin in a concentration of at least 5 µm, for example, 8 µm or more, the expression of Oct4 and Nanog decreased, whereas the expression of Pax7 increased. Accordingly, it was confirmed that the differentiation of the muscle stem cells into myoblasts could be induced in the culture medium supplemented with curcumin in the concentration of 5 µm or more.

### Experimental Example 1.2 Confirmation of bFGF-free curcumin culture medium on maintaining differentiation ability of muscle stem cells

5 % FBS and curcumin were added to the DMEM basal medium of Example 1, and effects of maintaining differentiation ability of muscle stem cells were confirmed.

In detail, first, bovine rump meat was treated with trypsin, and then primary-cultured to obtain bovine muscle stem cells. Afterwards, a culture medium supplemented with curcumin was prepared by varying the concentration of curcumin (0.1 µm, 1 µm, 5 µm, 8 µm, and 10 µm, respectively) in a DMEM basal medium supplemented with 5 % FBS, and the muscle stem cells were cultured through passage culture twice. In addition, the expression levels of Oct4 and Nanog as muscle stem cell markers and Pax7 as a myoblast marker in the culture medium were confirmed through qPCR, and the results are shown in FIGS. 6 to 8, respectively.

FIG. 6 is a graph showing the expression level of Oct4 as a muscle stem cell marker according to the concentration of curcumin in the bFGF-free curcumin culture medium.

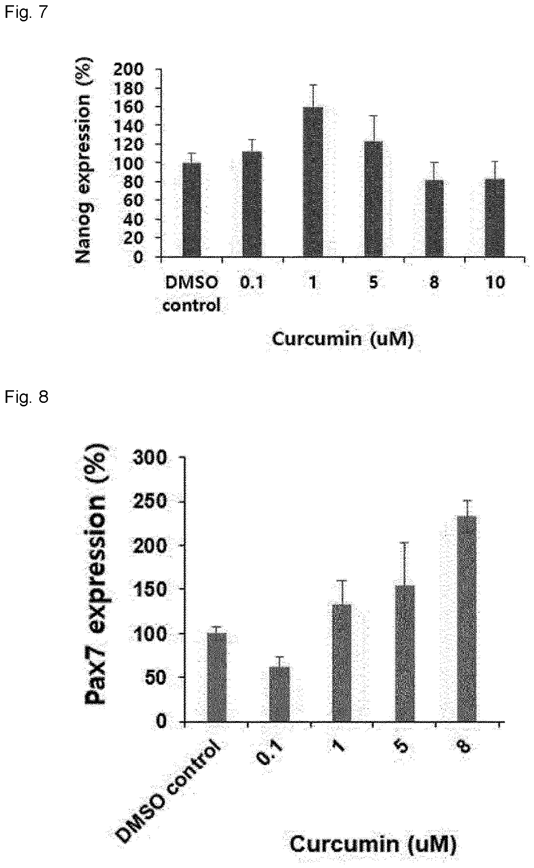

FIG. 7 is a graph showing the expression level of Nanog as a muscle stem cell marker according to the concentration of curcumin in the bFGF-free curcumin culture medium.

FIG. 8 is a graph showing the expression level of Pax7 as a myoblast marker according to a concentration of curcumin in the bFGF-free curcumin culture medium.

As shown in FIG. 6, the expression of Oct4 in the culture medium supplemented with curcumin in the concentration of 1 µm to 5 µm increased by at least about twice the expression of Oct4 in the curcumin-free culture medium control (i.e., DMSO control), confirming that the differentiation ability was effectively maintained. Also, it was confirmed that, when 8 µm of curcumin was added, the expression of Oct4 was significantly decreased.

As shown in FIG. 7, the expression of Nanog was maintained in the culture medium supplemented with curcumin, confirming that the differentiation ability of the muscle stem cells was effectively maintained. Also, it was confirmed that, when 8 µm of curcumin was added, the expression of Nanog was significantly decreased.

As shown in FIG. 8, it was confirmed that the expression of Pax7 increased in proportion to the concentration of curcumin in the culture medium supplemented with curcumin.

That is, it was confirmed that the expression of Oct4 and Nanog as muscle stem cell markers was effectively maintained even in the bFGF-free curcumin culture medium. Also, it was confirmed that, in the curcumin culture medium supplemented with 8 µm of curcumin, the expression of Oct4 and Nanog as muscle stem cell markers was significantly decreased, whereas the expression of Pax7 as a myoblast marker significantly increased. This means that the appropriate differentiation time may be chosen by controlling the concentration of curcumin, and the quality of in vitro meat may be improved by differentiating the muscle stem cells when necessary.

### Experimental Example 3. Confirmation of effects of glycine-containing medium on proliferation of muscle stem cells

### Experimental Example 3.1 Conformation of culture medium supplemented with glycine and bFGF on proliferation of muscle stem cells

5 % FBS, 8 ng/ml of bFGF, and 100 mM of glycine were added to the DMEM basal medium of Example 2, and effects of proliferating muscle stem cells were confirmed.

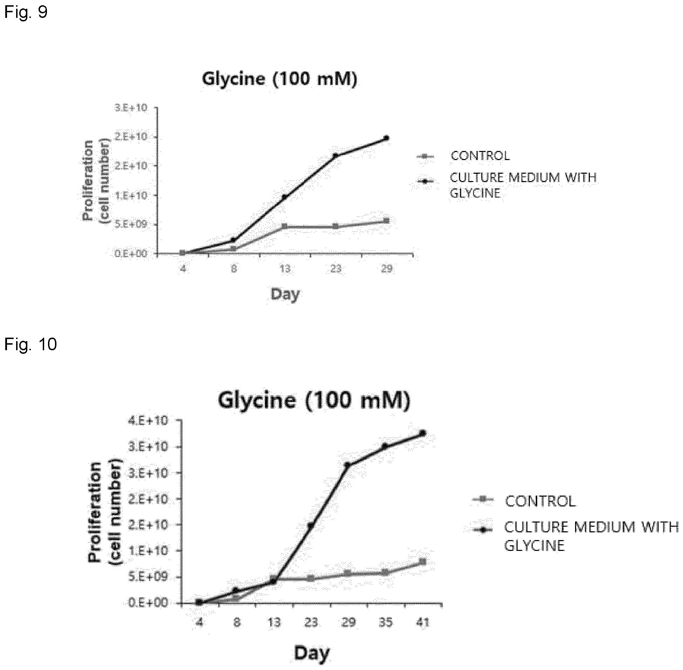

In detail, first, bovine rump meat was treated with trypsin, and then primary-cultured to obtain bovine muscle stem cells. Afterwards, the muscle stem cells were cultured in the DMEM basal medium supplemented with 5% FBS, 100 mM of glycine, and 8 ng/ml of bFGF, and the proliferation of the muscle stem cells was measured. Next, the results are shown in FIG. 9. As a control, a DMEM basal medium supplemented with 5 % FBS + 8 ng/ml of bFGF was used.

FIG. 9 is a graph showing the effects of the culture medium supplemented with glycine and bFGF on the proliferation of muscle stem cells.

As shown in FIG. 9, it was confirmed that, when the muscle cells were cultured for 29 days, the cell proliferation rate in the culture medium supplemented with 100 mM of glycine was increased by about 1.5 times or more compared to that in the basal medium.

### Experimental Example 3.2 Confirmation of bFGF-free glycine culture medium on proliferation of muscle stem cells

5 % FBS and 100 mM of glycine were added to the DMEM basal medium of Example 2, and effects of proliferating muscle stem cells were confirmed.

In detail, first, bovine rump meat was treated with trypsin, and then primary-cultured to obtain bovine muscle stem cells. Afterwards, the muscle stem cells were cultured in the DMEM basal medium supplemented with 5% FBS and 100 mM of glycine, and the proliferation of the muscle stem cells was measured. Next, the results are shown in FIG. 10. As a control, a DMEM basal medium supplemented with 5 % FBS was used.

FIG. 10 is a graph showing the effects of the bFGF-free glycine culture medium on the proliferation of muscle stem cells.

As shown in FIG. 10, it was confirmed that, when the muscle cells were cultured for 41 days, the cell proliferation rate in the culture medium supplemented with 100 mM of glycine was increased by about 4.1 times or more compared to that in the basal medium.

Referring to the results above, it was confirmed that, regardless of the presence of bFGF, glycine could act as a proliferation promoter for muscle stem cells.

### Experimental Example 4. Confirmation of effects of glycine-containing culture medium on maintaining differentiation ability of muscle stem cells

### Experimental Example 4.1 Conformation of culture medium with glycine and bFGF on maintaining differentiation ability of muscle stem cells

5 % FBS, 8 ng/ml of bFGF, and glycine were added to the DMEM basal medium of Example 2, and effects of maintaining differentiation ability of muscle stem cells were confirmed.

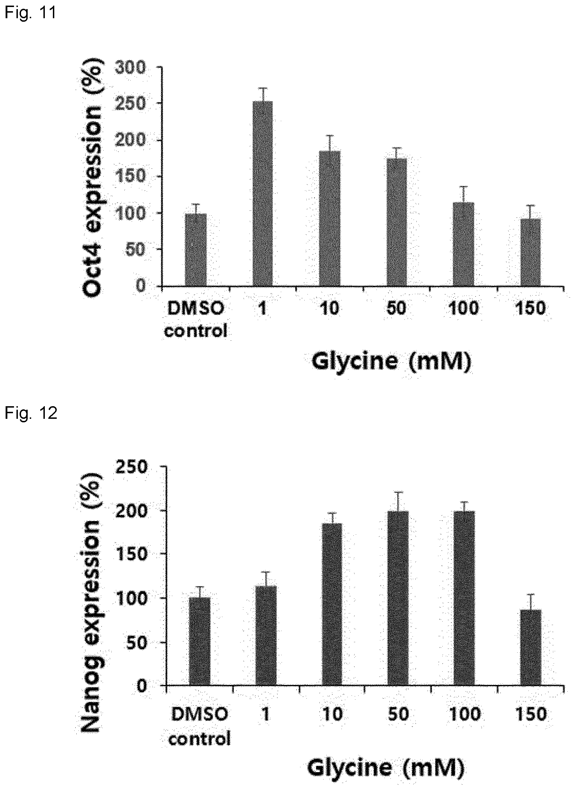

In detail, first, bovine rump meat was treated with trypsin, and then primary-cultured to obtain bovine muscle stem cells. Afterwards, a culture medium supplemented with glycine and bFGF was prepared by varying the concentration of glycine (1 mM, 10 mM, 50 mM, 100 mM, and 150 mM, respectively) in a DMEM basal medium supplemented with 5 % FBS and 8 ng/ml of bFGF, and the muscle stem cells were cultured through passage culture twice. In addition, the expression levels of Oct4 and Nanog as muscle stem cell markers and Pax7 as a myoblast marker in the culture medium were confirmed through qPCR, and the results are shown in FIGS. 11 to 13, respectively.

FIG. 11 is a graph showing the expression level of Oct4 as a muscle stem cell marker according to the concentration of glycine in the culture medium supplemented with glycine and bFGF.

FIG. 12 is a graph showing the expression level of Nanog as a muscle stem cell marker according to the concentration of glycine the culture medium supplemented with glycine and bFGF.

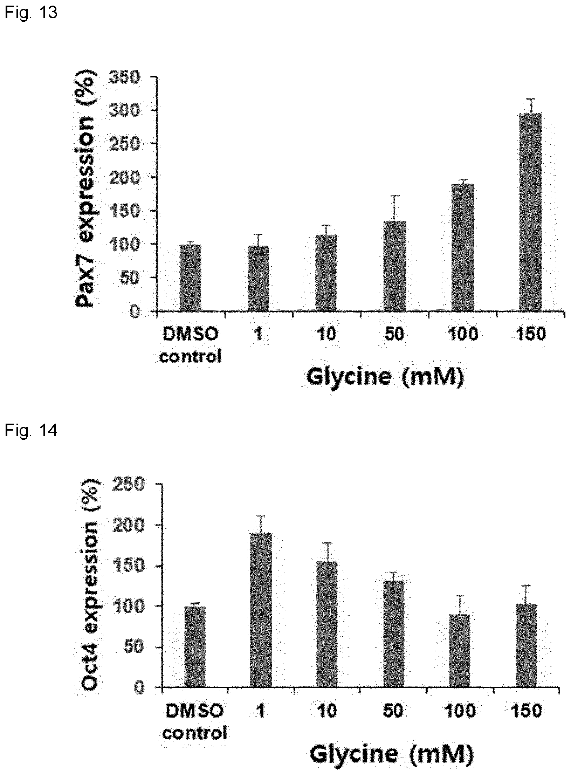

FIG. 13 is a graph showing the expression level of Pax7 as a myoblast marker according to a concentration of glycine in the culture medium supplemented with glycine and bFGF.

As shown in FIG. 11, the expression of Oct 4 increased in the culture medium supplemented with glycine and bFGF, confirming that the differentiation ability of the muscle stem cells was effectively maintained. In particular, it was confirmed that the expression of Oct4 in the culture medium supplemented with glycine in the concentration of 1 mM to 50 mM increased by about 2.3 times compared to the expression of Oct4 in the glycine-free culture medium control (i.e., DMSO control). Also, it was confirmed that, when the concentration of glycine was 100 mM or more, the expression of Oct4 significantly decreased.

As shown in FIG. 12, the expression of Nanog increased in the culture medium supplemented with glycine and bFGF, confirming that the differentiation ability of the muscle stem cells was effectively maintained. In particular, it was confirmed that the expression of Nanog in the culture medium supplemented with glycine in the concentration of 10 mM to 100 mM increased by about twice the expression of Oct4 in the glycine-free culture medium control (i.e., DMSO control). Also, it was confirmed that, when the concentration of glycine was 150 mM or more, the expression of Nanog significantly decreased.

As shown in FIG. 13, the expression of Pax7 increased in proportion to the concentration of glycine in the culture medium supplemented with glycine and bFGF.

Referring to the results above, it was confirmed that the differentiation ability of muscle stem cells could be maintained when the muscle stem cells were cultured in the culture medium supplemented with glycine. Also, it was confirmed that, in the culture medium supplemented with glycine in a concentration of at least 100 mM, for example, 150 mM or more, the expression of Oct4 and Nanog decreased, whereas the expression of Pax7 increased. Accordingly, it was confirmed that the differentiation of the muscle stem cells into myoblasts could be induced in the culture medium supplemented with glycine in a concentration of 100 mM or more, for example, 150 mM or more.

### Experimental Example 4.2 Confirmation of bFGF-free glycine culture medium on maintaining differentiation ability of muscle stem cells

5 % FBS and glycine were added to the DMEM basal medium of Example 2, and effects of maintaining differentiation ability of muscle stem cells were confirmed.

First, bovine rump meat was treated with trypsin, and then primary-cultured to obtain bovine muscle stem cells. Afterwards, a culture medium supplemented with glycine was prepared by varying the concentration of glycine (1 mM, 10 mM, 50 mM, 100 mM, and 150 mM, respectively) in a DMEM basal medium supplemented with 5 % FBS, and the muscle stem cells were cultured through passage culture twice. In addition, the expression levels of Oct4 and Nanog as muscle stem cell markers and Pax7 as a myoblast marker in the culture medium were confirmed through qPCR, and the results are shown in FIGS. 14 to 16, respectively.

FIG. 14 is a graph showing the expression level of Oct4 as a muscle stem cell marker according to the concentration of glycine in the bFGF-free glycine culture medium.

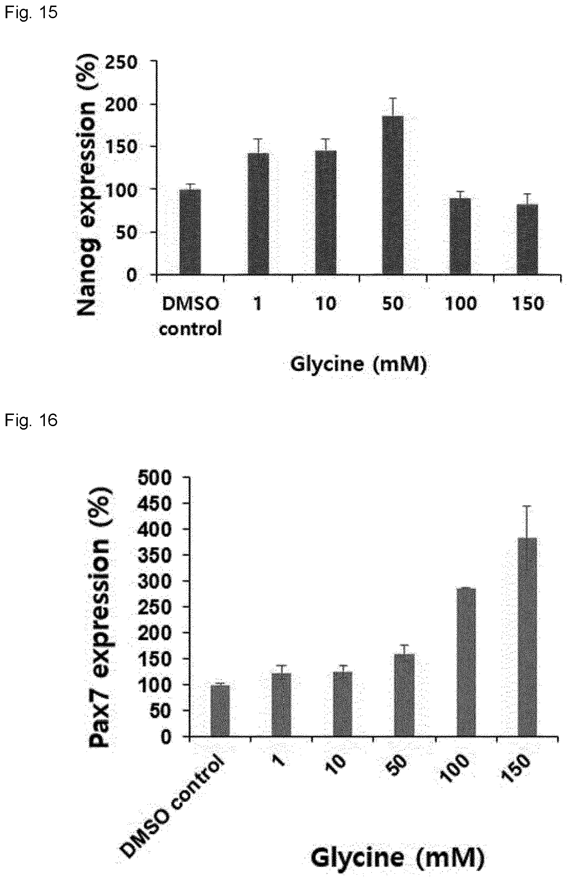

FIG. 15 is a graph showing the expression level of Nanog as a muscle stem cell marker according to the concentration of glycine in the bFGF-free glycine culture medium.

FIG. 16 is a graph showing the expression level of Pax7 as a myoblast marker according to the concentration of glycine in the bFGF-free glycine culture medium.

As shown in FIG. 14, the expression of Oct4 in the culture medium supplemented with glycine in the concentration of 1 mM to 50 mM increased by about twice the expression of Oct4 in the glycine-free culture medium control (i.e., DMSO control), confirming that the differentiation ability was effectively maintained. Also, it was confirmed that, when 100 mM of glycine was added, the expression of Oct4 was significantly decreased.

As shown in FIG. 15, the expression of Nanog in the culture medium supplemented with glycine in the concentration of 1 mM to 50 mM increased by about twice the expression of Oct4 in the glycine-free culture medium control (i.e., DMSO control), confirming that the differentiation ability was effectively maintained. Also, it was confirmed that, when 100 mM of glycine was added, the expression of Nanog was significantly decreased.

As shown in FIG. 16, the expression of Pax7 increased in proportion to the concentration of glycine in the culture medium supplemented with glycine. In particular, it was confirmed that the expression of Pax7 in the culture medium supplemented with glycine in the concentration of 100 mM or more increased by about 3 times or more compared to the expression of Pax7 in the glycine-free culture medium control (i.e., DMSO control).

That is, it was confirmed that the expression of Oct4 and Nanog as muscle stem cell markers was effectively maintained even in the bFGF-free glycine culture medium. Also, it was confirmed that, in the glycine culture medium supplemented with 100 mM of glycine, the expression of Oct4 and Nanog as muscle stem cell markers was significantly decreased, whereas the expression of Pax7 as a myoblast marker significantly increased. This means that the appropriate differentiation time may be chosen by controlling the concentration of glycine, and the quality of in vitro meat may be improved by differentiating the muscle stem cells when necessary.

### Experimental Example 5. Confirmation of effects of insulin-containing culture medium on proliferation of muscle stem cells

### Experimental Example 5.1 Conformation of culture medium supplemented with insulin and bFGF on proliferation of muscle stem cells

5 % FBS, 8 ng/ml of bFGF, and 10 µm of insulin were added to the DMEM basal medium of Example 3, and effects of proliferating muscle stem cells were confirmed.

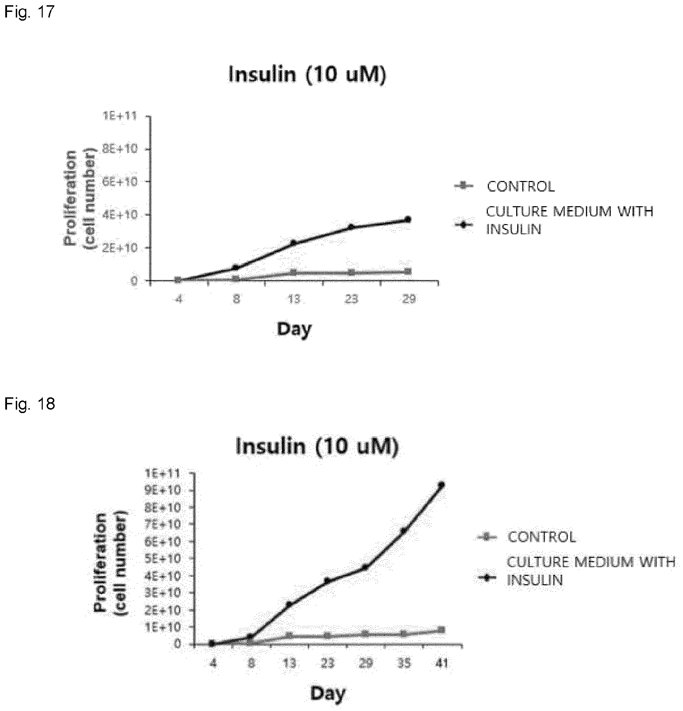

In detail, first, bovine rump meat was treated with trypsin, and then primary-cultured to obtain bovine muscle stem cells. Afterwards, the muscle stem cells were cultured in the DMEM basal medium supplemented with 5% FBS, 10 µm of insulin, and 8 ng/ml of bFGF, and the proliferation of the muscle stem cells was measured. Next, the results are shown in FIG. 17. As a control, a DMEM basal medium supplemented with 5 % FBS + 8 ng/ml of bFGF was used.

FIG. 17 is a graph showing the effects of the culture medium supplemented with insulin and bFGF on the proliferation of muscle stem cells.

As shown in FIG. 17, it was confirmed that, when the muscle cells were cultured for 29 days, the cell proliferation rate in the culture medium supplemented with 10 µm of insulin was increased by about 6 times or more compared to that in the basal medium.

### Experimental Example 5.2 Confirmation of bFGF-free insulin culture medium on proliferation of muscle stem cells

5 % FBS and 10 µm of curcumin were added to the DMEM basal medium of Example 3, and effects of proliferating muscle stem cells were confirmed.

In detail, first, bovine rump meat was treated with trypsin, and then primary-cultured to obtain bovine muscle stem cells. Afterwards, the muscle stem cells were cultured in the DMEM basal medium supplemented with 5% FBS and 10 µM of insulin, and the proliferation of the muscle stem cells was measured. Next, the results are shown in FIG. 18. As a control, a DMEM basal medium supplemented with 5 % FBS was used.

FIG. 18 is a graph showing the effects of the bFGF-free insulin culture medium on the proliferation of muscle stem cells.

As shown in FIG. 18, it was confirmed that, when the muscle cells were cultured for 41 days, the cell proliferation rate in the culture medium supplemented with 10 µm of insulin was increased by about 13 times or more compared to that in the basal medium.

Referring to the results above, it was confirmed that, regardless of the presence of bFGF, insulin could act as a proliferation promoter for muscle stem cells.

### Experimental Example 6. Confirmation of effects of insulin-containing culture medium on maintaining differentiation ability of muscle stem cells

### Experimental Example 6.1 Conformation of culture medium supplemented with insulin and bFGF on maintaining differentiation ability of muscle stem cells

5 % FBS, 8 ng/ml of bFGF, and insulin were added to the DMEM basal medium of Example 3, and effects of maintaining differentiation ability of muscle stem cells were confirmed.

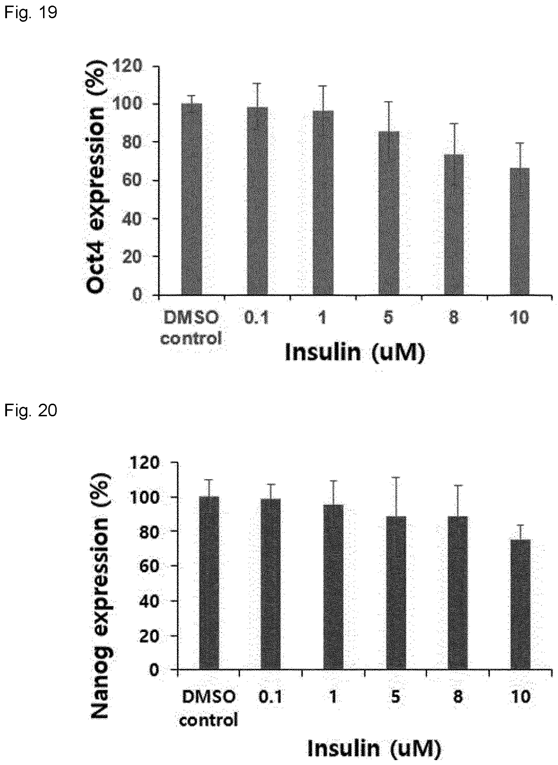

In detail, first, bovine rump meat was treated with trypsin, and then primary-cultured to obtain bovine muscle stem cells. Afterwards, a culture medium supplemented with insulin and bFGF was prepared by varying the concentration of insulin (0.1 µm, 1 µm, 5 µm, 8 µm, and 10 8 µm, respectively) in a DMEM basal medium supplemented with 5 % FBS and 8 ng/ml of bFGF, and the muscle stem cells were cultured through passage culture twice. In addition, the expression levels of Oct4 and Nanog as muscle stem cell markers and Pax7 as a myoblast marker in the culture medium were confirmed through qPCR, and the results are shown in FIGS. 19 to 21, respectively.

FIG. 19 is a graph showing the expression level of Oct4 as a muscle stem cell marker according to the concentration of insulin in the culture medium supplemented with insulin and bFGF.

FIG. 20 is a graph showing the expression level of Nanog as a muscle stem cell marker according to the concentration of insulin in the culture medium supplemented with insulin and bFGF.

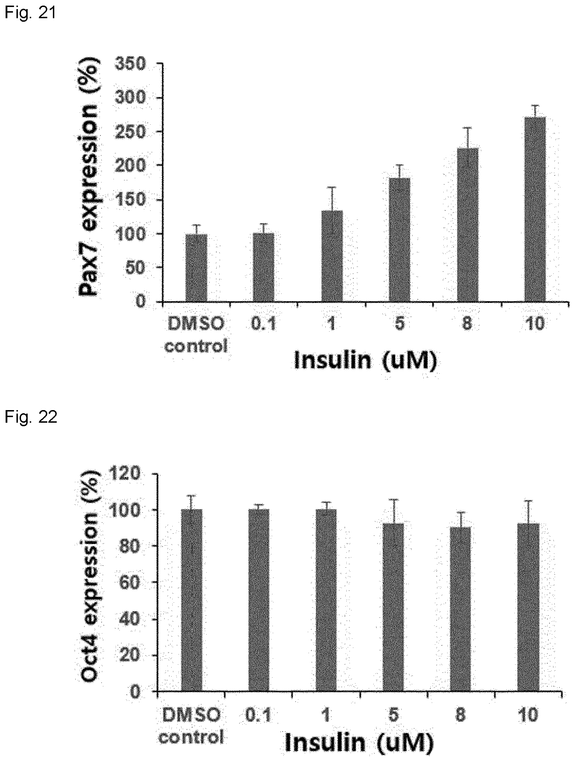

FIG. 21 is a graph showing the expression level of Pax7 as a myoblast marker according to the concentration of insulin in the culture medium supplemented with insulin and bFGF.

As shown in FIG. 19, the expression of Oct 4 increased in the culture medium supplemented with insulin and bFGF, confirming that the differentiation ability of the muscle stem cells was maintained. Also, it was confirmed that, when the concentration of insulin was 10 µm or more, the expression of Oct4 significantly decreased.

As shown in FIG. 20, the expression of Nanog increased in the culture medium supplemented with insulin and bFGF, confirming that the differentiation ability of the muscle stem cells was maintained. Also, it was confirmed that, when the concentration of insulin was 10 µm or more, the expression of Nanog significantly decreased.

As shown in FIG. 21, the expression of Pax7 increased in proportion to the concentration of insulin in the culture medium supplemented with insulin and bFGF.

Referring to the results above, it was confirmed that the differentiation ability of muscle stem cells could be maintained when the muscle stem cells were cultured in the culture medium supplemented with insulin. Also, it was confirmed that, in the culture medium supplemented with insulin in the concentration of at least 10 µm, the expression of Oct4 and Nanog decreased, whereas the expression of Pax7 increased. Accordingly, it was confirmed that the differentiation of the muscle stem cells into myoblasts could be induced in the culture medium supplemented with insulin in the concentration of 10 µm or more.

### Experimental Example 6.2 Confirmation of bFGF-free insulin culture medium on maintaining differentiation ability of muscle stem cells

5 % FBS and insulin were added to the DMEM basal medium of Example 3, and effects of maintaining differentiation ability of muscle stem cells were confirmed.

In detail, first, bovine rump meat was treated with trypsin, and then primary-cultured to obtain bovine muscle stem cells. Afterwards, a culture medium supplemented with insulin was prepared by varying the concentration of insulin (0.1 µm, 1 µm, 5 µm, 8 µm, and 10 µm, respectively) in a DMEM basal medium supplemented with 5 % FBS, and the muscle stem cells were cultured through passage culture twice. In addition, the expression levels of Oct4 and Nanog as muscle stem cell markers and Pax7 as a myoblast marker in the culture medium were confirmed through qPCR, and the results are shown in FIGS. 22 to 24, respectively.

FIG. 22 is a graph showing the expression level of Oct4 as a muscle stem cell marker according to the concentration of insulin in the bFGF-free insulin culture medium.

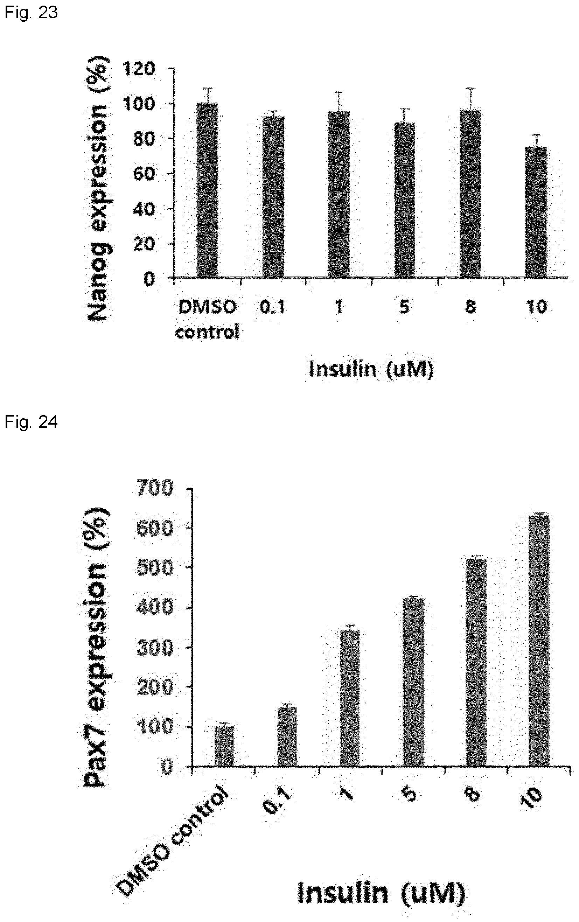

FIG. 23 is a graph showing the expression level of Nanog as a muscle stem cell marker according to the concentration of insulin in the bFGF-free insulin culture medium.

FIG. 24 is a graph showing the expression level of Pax7 as a myoblast marker according to the concentration of insulin in the bFGF-free insulin culture medium.

As shown in FIG. 22, the expression of Oct 4 increased in the culture medium supplemented with insulin, confirming that the differentiation ability of the muscle stem cells was maintained.

As shown in FIG. 23, the expression of Nanog was maintained in the culture medium supplemented with insulin, confirming that the differentiation ability of the muscle stem cells was effectively maintained. Also, it was confirmed that, when 10 µm of insulin was added, the expression of Nanog was significantly decreased.

As shown in FIG. 24, the expression of Pax7 increased in proportion to the concentration of insulin in the culture medium supplemented with insulin. In particular, it was confirmed that the expression of Pax7 in the culture medium supplemented with glycine in the concentration of 10 µM increased by about 6 times or more compared to the expression of Pax7 in the insulin-free culture medium control (i.e., DMSO control).

That is, it was confirmed that the expression of Oct4 and Nanog as muscle stem cell markers was effectively maintained even in the bFGF-free insulin culture medium. Also, it was confirmed that, in the insulin culture medium supplemented with 10 µm of insulin, the expression of Nanog as a muscle stem cell marker was significantly decreased, whereas the expression of Pax7 as a myoblast marker significantly increased. This means that the appropriate differentiation time may be chosen by controlling the concentration of insulin, and the quality of in vitro meat may be improved by differentiating the muscle stem cells when necessary.

## Claims

1. A culture medium composition for culturing muscle stem cells, comprising turmeric, glycine, or insulin.

2. The culture medium composition of claim 1, wherein the turmeric is a turmeric extract or a fraction thereof.

3. The culture medium composition of claim 2, wherein the turmeric extract or the fraction thereof comprises curcumin.

4. The culture medium composition of claim 3, wherein the curcumin is comprised in a concentration of 0.01 µM to 100 µM.

5. The culture medium composition of claim 3, wherein the curcumin is comprised in a concentration of at least 8 µM.

6. The culture medium composition of claim 1, wherein the glycine is comprised in a concentration of 0.1 mM to 1,000 mM.

7. The culture medium composition of claim 1, wherein the glycine is comprised in a concentration of at least 100 mM.

8. The culture medium composition of claim 1, wherein the insulin is comprised in a concentration of 0.01 µM to 100 µM.

9. The culture medium composition of claim 1, wherein the insulin is comprised in a concentration of at least 10 µM.

10. The culture medium composition of claim 1, wherein the muscle stem cells are mammalian stem cells.

11. The culture medium composition of claim 1, wherein the medium composition is for maintaining the muscle stem cells in an undifferentiated state.

12. The culture medium composition of claim 1, wherein the culture medium is bFGF-free.

13. A method of culturing muscle stem cells, the method comprising culturing muscle stem cells isolated from a mammal in a culture medium for culturing muscle stem cells, the culture medium comprising turmeric, glycine, or insulin.

14. The method of claim 13, wherein the culturing is passage culture.

15. A method of preparing in vitro meat, the method comprising preparing in vitro meat by treating muscle stem cells derived from a non-human animal with a culture medium composition for culturing muscle stem cells, the culture medium composition comprising turmeric, glycine, or insulin.

16. In vitro meat prepared by the method of claim 15.

17. Use of turmeric, glycine, or insulin for culturing muscle stem cells.
